Europäisches Patentamt

European Patent Office

Office européen des brevets

(11) Publication number: **0 206 625 B1**

(12) **EUROPEAN PATENT SPECIFICATION**

(45) Date of publication of patent specification: **30.09.92**

(51) Int. Cl.⁵: **A61K 33/00**, A61K 31/60, A61K 31/19, A61K 31/29, A61K 33/24

(21) Application number: **86304407.9**

(22) Date of filing: **10.06.86**

Divisional application 91114034.1 filed on 10/06/86.

The file contains technical information submitted after the application was filed and not included in this specification

(54) **Compositions for the treatment of gastrointestinal disorders.**

(30) Priority: **13.06.85 US 744841**

(43) Date of publication of application:
**30.12.86 Bulletin 86/52**

(45) Publication of the grant of the patent:
**30.09.92 Bulletin 92/40**

(84) Designated Contracting States:
**AT BE CH DE FR GB IT LI LU NL SE**

(56) References cited:
**DE-A- 1 963 496**
**FR-M- 6 531**
**US-A- 4 016 268**
**US-A- 4 118 480**

**CHEMICAL ABSTRACTS, vol. 94, 1981, Columbus, OH (US); S.H.LORBER, p. 93, no. 168025b***

**UNLISTED DRUGS, vol. 22, no. 11, November 1970, Chatham,New York, NY (US); "Aldefur", p. 163b***

(73) Proprietor: **Marshall, Barry James, Dr.**
**940 Stanley Drive Earlysville**
**Virginia 22936(US)**

(72) Inventor: **Marshall, Barry James, Dr.**
**940 Stanley Drive Earlysville**
**Virginia 22936(US)**

(74) Representative: **Brooks, Maxim Courtney et al**
**Procter & Gamble (NTC) Limited Whitley Road Longbenton**
**Newcastle-upon-Tyne NE12 9TS(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

WORLD CONGRESS OF GASTROENTEROL-
OGY, 26-31 August 1990, Sidney (AU);
"Heliobacter pylori: Casual agent in peptic
ulcerdisease?", pp. 36-45*

GASTROENTEROLOGY, vol. 94, 1988; RAUWS
et al., pp. 33-40*

GASTROENTEROLOGY, vol. 92, no. 5, 1987;
MARSHALL et al., p. 1518*

GASTROENTEROLOGY, vol. 92, no. 5/2, 1987;
BORODY et al., p. 1324*

MED. J. AUSTRALIA, vol. 146, 1987; BORODY
et al., pp. 450-451*

CHEMICAL ABSTRACTS, vol. 98, 1983, Co-
lumbus, OH (US); S.B.COGHILL et al., p. 15,
no. 172475y*

## Description

This invention relates to compositions for the treatment of gastrointestinal disorders in humans and other animals.

Factors adversely affecting the function of the gastrointestinal system in humans are exceedingly varied in their nature. Such disorders may arise in the upper or lower gastrointestinal tracts or both. There is a broad range of causes of gastrointestinal disorders, including genetic, physiological, environmental, and psychogenic factors. Accordingly, the diagnosis and management of these disorders can be exceptionally difficult. A detailed discussion of gastrointestinal tract functions, disorders, causes, and treatments can be found in Spiro, Clinical Gastroenterology (3d. edition 1983).

Among the chronic disorders of the upper gastrointestinal tract are those which fall under the general categories of gastritis and peptic ulcer disease. (The upper gastrointestinal tract is generally defined as including the esophagus, the stomach, the duodenum, the jejunum, and ilium.) Gastritis is, by definition, typified by an inflammation of the stomach mucosa. In practice, though, the disorder is manifested by a broad range of poorly-defined, and heretofore inadequately treated, symptoms such as indigestion, "heart burn", dyspepsia and excessive eructation. A general discussion of gastritis appears in B. J. Marshall and J. R. Warren, "Unidentified Curved Bacilli in the Stomach of Patients with Gastritis and Peptic Ulceration", The Lancet, 1311-1315 (1984), and in R. Greenlaw, et al., "Gastroduodenitis, A Broader Concept of Peptic Ulcer Disease", 25 Digestive Diseases and Sciences 660-672 (1980).

Peptic ulcers are lesions of the gastrointestinal tract lining, characterized by loss of tissue due to the action of digestive acids and pepsin. It has been generally held that peptic ulcers are caused either by gastric hypersecretion, or (more often) by decreased resistance of the gastric lining to digestive acids and pepsin. The medical literature is replete with methods for treating ulcers, including modification of the diet, surgical removal of the lesions, and the use of drugs. Such drugs include: antacids, which serve to counteract excess gastric secretions; anticholinergics, which reduce acid secretion; $H_2$ antagonists, which also block the release of gastric acids; prostaglandins, which increase the resistance of the gastric lining to digestive fluids, and may also inhibit acid secretion; prokinetic agents, which enhance gastrointestinal tract motility; and compositions which form protective barriers over gastric lesions. Prescription and non-prescription drug therapies are generally described in Garnet, "Antacid Products", Handbook of Non-prescription Drugs, Chapter 3 (7th edition, 1982). One group of drugs which are thought to be effective due to coating of ulcer sites and forming protective barriers is the bismuth-containing drugs. See, for example, Koo, et al., "Selective Coating of Gastric Ulcers by Tripotassium Dicitrato Bismuthate in the Rat", 82 Gastroenterology 864-870 (1982).

Regardless of the particular drug composition used in treating gastrointestinal disorders, such as peptic ulcer disease, the treatment is often imprecise and incomplete. Actual "cures", i.e., successful treatment resulting in total remission of disease, are very often not effected. See, A. J. McLean, et al., "Cyto-protective Agents and Ulcer Relapse", 142 The Medical Journal of Australia, Special Supplement S25-S28 (1985). Furthermore, many conventional treatments may render subjects hypochlorhydric (i.e., with low levels of hydrochloric acid in the stomach) which may predispose them to other disorders, e.g., gastrointestinal infections, halitosis, and gastric carcinomas.

It has now been discovered that certain compositions of bismuth and antimicrobials are effective for the treatment of gastrointestinal disorders. In particular, as compared to the art, these compositions cure, or afford lower relapse rates of, gastritis and peptic ulcer disease.

These compositions also afford other benefits in the treatment and management of subjects having gastrointestinal diseases, such as in not rendering treated subjects hypochlorohydric.

According to the present invention, there is provided a composition for the treatment of gastrointestinal disorders in a human or other animal subject, comprising:

(a) from 50 mg to 5,000 mg of bismuth; and

(b) from 100 mg to 10,000 mg of an antimicrobial selected from gentamicin, neomycin, kanamycin, streptomycin erythromycin, clindamycin, rifampin, penecillin G, penicillin V ampicillin, amoxycillin, bacitracin, polymyxin, tetracycline, chlortetracycline, oxytetracycline, doxycyline, cephalexin, cephalothin, chloramphenicol, sulfonamides, nitrofurazone, nitrofurantoin, furozolidone, metronidazole, tinidazole, nimorazole, and mixtures thereof.

Specific compounds and compositions to be used in the compositions of the present invention must be pharmaceutically-acceptable. As used herein, such a 'pharmaceutically-acceptable' component is one which is suitable for use with humans and/or animals without undue adverse side effects (such as toxicity, irritation, and allergic response) commensurate with a reasonable benefit/risk ratio. Further, as used herein, the term "safe and effective amount" refers to the quantity of a component which is sufficient to yield a desired therapeutic response without undue adverse effects (such as toxicity, irritation, or allergic response) commensurate with a reasonable benefit/risk ratio when used in the manner of this invention. The specific "safe and effective amount" will, obviously, vary with such factors as the particular condition that is being treated, the severity of the condition, the duration of the treatment, the physical condition of the patient, the nature of concurrent therapy (if any), and the specific formulations employed in the present invention.

As used herein, "gastrointestinal disorder" encompasses any disease or other disorder of the upper gastrointestinal tract of a human or lower animal. Such gastrointestinal disorders include, for example: disorders not manifested by presence of ulcerations in the gastric mucosa (herein "non-ulcerative gastrointestinal disorder"), including chronic or atrophic gastritis, non-ulcer dyspepsia, esophogeal reflux disease and gastric motility disorders; and "peptic ulcer disease", i.e., gastric, duodenal and jejunal ulcers. In particular, "gastrointestinal disorder" refers to such disorders of the upper gastrointestinal tract caused or mediated by bacteria, including campylobacter-like organisms (herein "CLO"), e.g., Campylobacter pyloridis. Such CLO include those described in J. R. Warren and B. J. Marshall, "Unidentified Curved Bacilli on Gastric Epithelium in Active Chronic Gastritis", The Lancet 1273-1275 (1983) and G. Kasper and N. Dickgiesser, "Isolation from Gastric Epithelium of Campylobacter-like Bacteria that are Distinct from 'Campylobacter Pyloridis'", The Lancet 111-112 (1985).

As used herein, "administering" refers to any method which, in sound medical practice, delivers the compositions used in this invention to the subject to be treated in such a manner so as to be effective in the treatment of the gastrointestinal disorder.

The bismuth is preferably administered as a pharmaceutically-acceptable salt. Such bismuth salts include, for example, bismuth aluminate, bismuth subcarbonate, bismuth subcitrate, bismuth citrate, tripotassium dicitrato bismuthate, bismuth subgalate, bismuth subnitrate, bismuth tartrate, bismuth subsalicylate, and mixtures thereof. Bismuth citrate, bismuth subcitrate, tripotassium dicitrato bismuthate, bismuth tartrate, bismuth subsalicylate, and mixtures thereof are preferred bismuth salts for use in this invention. The bismuth useful herein may be administered alone, or in combination with other pharmaceutically-acceptable components, in a bismuth-containing composition. A variety of such compositions containing bismuth salts are commercially-available, including, for example, DeNol (RTM), containing tripotassium dicitrato bismuthate (sold by Gist-Brocades N.V.), Noralac (RTM), containing bismuth aluminate, alginic acid, and magnesium carbonate (manufactured by North American Pharmaceuticals), Roter (RTM) bismuth, containing bismuth subnitrate (sold by Roter Laboratories), Fensobar Polvo (RTM), containing bismuth subcarbonate among other materials (manufactured by USV Pharmaceutical Corporation), and Pepto-Bismol (RTM), containing bismuth subsalicylate (sold by The Procter & Gamble Company).

A wide variety of antimicrobials are useful in this invention. As used herein, such "antimicrobials" refer to a naturally-occurring, synthetic or semi-synthetic antibiotic compound or composition, or mixture thereof, which is safe for human use as used in the processes of this invention, and is effective in killing or substantially inhibiting the growth of CLO when used in the compositions of this invention. Suitable antibiotics can be generally classified by chemical composition, into the following principal groups: aminoglycosides selected from gentamicin neomycin, kanamycin, and streptomycin; macrolides selected from erythromycin, clindamycin, and rifampin; penicillins selected from penicillin G, pencillin V, ampicillin and amoxycillin; polypetides selected from bacitracin and polymyxin; tetracyclines, selected from tetracycline, chlortetracycline, oxytetracycline, and doxycycline; cephalosporins selected from cephalexin and cephalothin; and the miscellaneous antibiotics chloramphenicol and clindamycin. These antibiotics can be generally said to function in one of four ways: inhibition of cell walls synthesis, alteration of cell wall permeability, inhibition of protein synthesis, or inhibition of nucleic acid synthesis.

Other antimicrobials useful herein are the sulfonamides; nitrofurans selected from nitrofurazone, nitrofurantoin, and furozolidone; and metronidazole, tinidazole, and nimorazole. Antimicrobials among those useful herein are described in the following publications: Remington's Pharmaceutical Sciences (15th edition 1975); F. H. Meyers, et al., Review of Medical Pharmacology (7th edition 1980); Gaddum's Pharmocology - (8th edition 1978); and A. Goodman, A. G. Goodman and L. S. Gilman, The Pharmacological Basis of Therapeutics (6th edition 1980).

While any of these antimicrobials may be used, penicillin, erythromycin, doxycycline, metronidazole, tinidazole, amoxycillin, ampicillin, and nitrofurantoin are among the preferred antimicrobials for use in the present invention. Preferably, a sample of CLO is obtained from the stomach of the subject to be treated, as by biopsy, aspiration, or by other suitable method, and the organism cultured and tested for sensitivity to the various antimicrobials useful herein. Preferably such sensitivity testing is by determination of the relative minimum inhibitory concentrations of the antimicrobials using broth or plate dilution techniques. The antimicrobial found to be most effective against the cultured bacteria (i.e., effective at the lowest minimum inhibitory concentration) is then selected for use in this invention.

The present invention thus provides compositions, for the treatment of gastrointestinal disorders, comprising:

(a) from 50 mg to 5000 mg of bismuth; and

(b) from 100 mg to 10,000 mg of the herein specified antimicrobial.

Preferably, the bismuth salt is present at a level of from 250 mg to about 1000 mg. Also preferably, the antimicrobial is present at a level of from 100 mg to 1000 mg.

The compositions of the present invention may contain optional components which affect the physical and therapeutic characteristics of the present compositions. In particular, a variety of pharmaceutically-acceptable carriers and excipients may be included, depending upon the particular dosage form to be used. Various oral dosage forms can be used, including such solid forms as tablets, capsules, granules and bulk powders. Tablets can be compressed, tablet triturates, enteric-coated, sugar-coated, film-coated or multiple compressed, containing suitable binders, lubricants, diluents, disintegrating agents, coloring agents, flavoring agents, flow-inducing agents and melting agents. Liquid oral dosage forms include aqueous solutions, emulsions, suspensions, solutions and/or suspensions reconstituted from non-effervescent granules and effervescent preparations reconstituted from effervescent granules, containing suitable solvents, preservatives, emulsifying agents, suspending agents, diluents, sweeteners, melting agents, coloring, and flavoring agents.

Specific examples of pharmaceutically-acceptable carriers and excipients that may be used to formulate oral dosage forms of the present invention are described in US-A-3,903,297, Robert, issued September 2, 1975, Techniques and compositions for making dosage forms useful herein are described in the following references; 7 Modern Pharmaceutics, Chapters 9 and 10 (Banker and Rhodes, editors, 1979); an Lieberman, et a., Pharmaceutical Dosage Forms: Tablets (1981); and Ansel, Introduction to Pharmaceutical Dosage Forms (2d edition, 1976).

Care must be taken in avoiding any interactions between the bismuth compound or composition and the particular antimicrobial used in these compositions. For example, the presence of bismuth is known to adversely affect the efficacy of the tetracyclines. Accordingly, in preferred compositions of this invention, the antimicrobial is physically separated from the bismuth, in such a manner so that the antimicrobial and the bismuth are not simultaneously dissolved in the stomach. Hence, a preferred composition of this invention comprises a capsule containing bismuth particles and enterically-coated antimicrobial particles.

The compositions of this invention may be used by administering the composition from 1 to 7 times, per day, for from 3 to 21 days. The specific frequency of administration will depend upon such factors as the specific bismuth compound or composition and antimicrobial used, the levels at which the components are incorporated in the composition, the nature and severity of the condition to be treated, and the nature of any concurrent therapy (if any). Suitable methods for detecting CLO in the upper gastrointestinal tract of human or other animal subjects. include gram stains of gastric tissues (obtained, for example, by biopsy), serologic tests to detect the presence of antibodies to the organisms, tests of body fluids to detect the presence of metabolites of the organisms, silver or other specially stained tissue sections of tissues, and detection of urease enzyme in the stomach of the subject.

One preferred method of detection is the detection of urease enzyme (urea amidohydrolase) in the stomach of the human or lower animal having a gastrointestinal disorder. Such a detection step may include, for example, obtaining a sample of gastric fluid (e.g., from a gastric tube or vomitus) or of gastric mucosa (e.g., by biopsy) and analyzing the material for the presence of urease enzyme. One such method for the diagnosis of gastrointestinal disorder involves obtaining a sample of gastric mucosa and placing said sample into a composition which comprises:

a) urea, at a concentration of from about 10 to about 40 g/$\ell$;

b) a bactericide, at a concentration of from about 1 to about 5 g/$\ell$;

c) an indicator having a p$K_a$ of from about 6.5 to about 8.5, at an effective concentration; and

d) water;

wherein said composition has a pH of from about 5.0 to about 6.5 and said pH is at least one pH unit lower than the p$K_a$ of said indicator. Preferably, the composition contains a gelling agent, such as a non-nutritive agar, at a concentration of from about 5 to about 50 g/$\ell$. Typically, the indicator is present at a concentration of from about 20 to about 100 mg/$\ell$(As used herein, all concentrations are by weight of component per total volume of composition.) A change in the color of the composition indicates the presence of urease enzyme, and the presence of a gastrointestinal disorder.

The following examples illustrate the compositions of the present invention.

EXAMPLE I

A composition, according to the present invention, is made comprising:

| bismuth subsalicylate | 0.5g |
| penicillin | 0.5g |
| magnesium stearate | 0.4g |

The composition is made by simple admixture of the components and pressed into tablets using conventional methods. The composition is then administered to a human subject having peptic ulcer disease, three times daily for fourteen days. The subject, after treatment, is endoscoped, revealing healing of the peptic ulcer lesion, and histologic absence of active chronic gastritis and of CLO.

## Claims

1. A composition for the treatment of gastrointestinal disorders in a human or other animal subject, comprising:
   (a) from 50 mg to 5,000 mg of bismuth; and
   (b) from 100 mg to 10,000 mg of an antimicrobial selected from gentamicin, neomycin, kanamycin, streptomycin erythromycin, clindamycin, rifampin, penecillin G, penicillin V ampicillin, amoxycillin, bacitracin, polymyxin, tetracycline, chlortetracycline, oxytetracycline, doxycyline, cephalexin, cephalothin, chloramphenicol, sulfonamides, nitrofurazone, nitrofurantoin, furozolidone, metronidazole, tinidazole, nimorazole, and mixtures thereof.

2. A composition according to claim 1 comprising a bismuth compound selected from bismuth aluminate, bismuth subcarbonate, bismuth subcitrate, bismuth citrate, tripotassium dicitrato bismuthate, bismuth subgalate, bismuth subnitrate, bismuth tartrate, bismuth subsalicylate, and mixtures thereof.

## Patentansprüche

1. Zusammensetzung zur Behandlung von gastrointestinalen Störungen in einem menschlichen oder anderen tierischen Subjekt, umfassend:
   (a) von 50 mg bis 5.000 mg Bismuth; und
   (b) von 100 bis 10.000 mg eines antimikrobiellen Mittels ausgewählt unter Gentamicin, Neomycin, Kanamycin, Streptomycin, Erythromycin, Clindamycin, Rifampin, Penicillin G, Penicillin V, Ampicillin, Amoxycillin, Bacitracin, Polymyxin, Tetracyclin, Chlortetracyclin, Oxytetracyclin, Doxycylin, Cephalexin, Cephalothin, Chloramphenicol, Sulfonamiden, Nitrofurazon, Nitrofurantoin, Furozolidon, Metronidazol, Tinidazol, Nimorazol und deren Gemischen.

2. Zusammensetzung nach Anspruch 1, umfassend eine unter Bismuthaluminat, Bismuthsubcarbonat, Bismuthsubcitrat, Bismuthcitrat, Trikaliumdicitratobismuthat, Bismuthsubgalat, Bismuthsubnitrat, Bismuthtartrat, Bismuthsubsalicylat und deren Gemischen ausgewählte Bismuthverbindung.

## Revendications

1. Composition pour le traitement d'affections gastro-intestinales chez un être humain ou un autre sujet animal, la composition comprenant :
   (a) de 50 mg à 5 000 mg de bismuth ; et
   (b) de 100 mg à 10 000 mg d'un antimicrobien, choisi parmi la gentamicine, la néomycine, la kanamycine, la streptomycine, l'érythromycine, la clindamycine, la rifampine, la pénicilline G, la pénicilline V, l'ampicilline, l'amoxycilline, la bacitracine, la polymyxine, la tétracycline, la chlorotétracycline, l'oxytétracycline, la doxycycline, la céphalexine, la céphalothine, le chloramphénicol, les sulfamides , la nitrofurazone, la nitrofurantoïne, la furozolidone, le métronidazole, le tinidazole, le nimorazole et leurs mélanges.

2. Composition selon la revendication 1, comprenant un composé du bismuth choisi parmi l'aluminate de bismuth, le sous-carbonate de bismuth, le sous-citrate de bismuth, le citrate de bismuth le dicitratobismuthate tripotassique, le sous-galate de bismuth, le sous-nitrate de bismuth le tartrate de bismuth, le sous-salicylate de bismuth, et leurs mélanges.